# EUROPÄISCHE PATENTSCHRIFT

(11) **EP 2 548 382 B1**
(45) Veröffentlichungstag und Bekanntmachung des Hinweises auf die Patenterteilung: **06.08.2014**
(21) Anmeldenummer: 11709674.3
(22) Anmeldetag: 10.03.2011
(51) Int. Cl.: H04R 25/00

(54) **VERFAHREN ZUM TEST DES SPRACHVERSTEHENS EINER MIT EINEM HÖRHILFEGERÄT VERSORGTEN PERSON**
METHOD FOR TESTING SPEECH COMPREHENSION OF A PERSON ASSISTED BY A HEARING AID
MÉTHODE POUR TESTER LA COMPRÉHENSION DE LANGUE D'UNE PERSON ASSISTÉE D'UNE PROTÈSE AUDITIVE

(30) Priorität: 18.03.2010 DE 102010011950
(43) Veröffentlichungstag der Anmeldung: 23.01.2013
(73) Patentinhaber: Siemens Medical Instruments Pte. Ltd., Singapore 139959 (SG)
(72) Erfinder: BELLANOVA, Martina, 91058 Erlangen (DE); LATZEL, Matthias, 91330 Eggolsheim (DE)
(74) Vertreter: Maier, Daniel Oliver
(86) Internationale Anmeldenummer: PCT/EP2011/053600
(87) Internationale Veröffentlichungsnummer: WO 2011/113741

(56) Entgegenhaltungen:
- US-A1- 2005 027 537
- US-A1- 2006 093 172
- MICHAEL BORETZKI ET AL: "The Benefits of Nonlinear Frequency Compression for People with Mild Hearing Loss", INTERNET CITATION, 23. November 2009 (2009-11-23), Seiten 1-7, XP009147947, Gefunden im Internet: URL:http://www.phonak.com/content/dam/phon ak/b2b/C_M_tools/Library/Features/SoundRec over/en/NLFC_and_mild_HL_AudiologyOnline_N ov09.pdf [gefunden am 2011-05-05]

## Beschreibung

Die Erfindung betrifft ein Verfahren zum Test des Sprachverstehens einer mit einem Hörhilfegerät versorgten Person.

Hörhilfegeräte können dazu beitragen, dass eine Person mit einer gegenüber einem Normalhörenden verminderter Hörwahrnehmung seine Umwelt ähnlich wie der Normalhörende wahrnimmt. Dies gelingt jedoch nur mit einem qualitativ hochwertigen Hörhilfegerät, das zudem exakt an den individuellen Hörverlust der Person angepasst sein muss. Insbesondere ist es für Schwerhörige wichtig, sich mit anderen Personen unterhalten zu können und demnach Sprache zu verstehen. Es ist daher sinnvoll und notwendig, die Qualität eines Hörhilfegerätes und/oder die Qualität der Anpassung besonders in Bezug auf Sprachsignale zu prüfen.

Hörhilfegeräte werden bereits während der Entwicklung oder nach der Fertigung regelmäßig geprüft. Insbesondere wird auch getestet, inwieweit bestimmte Algorithmen, beispielsweise zur Kompression, zur Frequenzkompression, zur Störgeräuschunterdrückung etc. Einfluss auf die Sprachverständlichkeit einer mit dem Hörhilfegerät versorgten Person haben. Sprachverständlichkeitstests können aber auch während oder nach der Anpassung eines Hörhilfegerätes sinnvoll durchgeführt werden.

Aus der Druckschrift US 2005/0027537 A1 ist ein Verfahren zum Test und zur Optimierung von Hörhilfegeräten bekannt, bei dem ein mit einem Hörhilfegerät versorgter Benutzer sinnlose Silben dargeboten bekommt. In Abhängigkeit davon, ob die dargebotenen Silben richtig verstanden werden, werden einstellbare Parameter des Hörhilfegerätes neu eingestellt.

Ein Dokument von Michael Boretzki et al. ("The Benefits of Nonlinear Frequency Compression for People with Mild Hearing Loss", http://www.phonak.com/content/dam/phonak/b2b/C_M_tools/Librar y/Features/SoundRecover/en/NLFC and_mild_HL_AidiologyOnline_N ov09.pdf) zeigt einen adaptiven Logatomtest, bei welchem sich die Anpassung des Tests auf den Laustärkepegel eines gleichbleibenden Logatoms beschränkt.

Aufgabe der vorliegenden Erfindung ist es, ein Verfahren zum Test von Hörhilfegeräten, insbesondere imn Hinblick auf deren Auswirkung auf das Sprachverstehen, zu verbessern.

Diese Aufgabe wird durch ein Verfahren mit den Verfahrensschritten gemäß Patentanspruch 1 gelöst.

Zur Durchführung des Verfahrens wird ein Testsystem bereitgestellt. Dieses umfasst eine Steuereinheit zur Steuerung des Tests. Dabei erfolgt zunächst eine Auswahl eines sinnlosen Wortes, vorzugsweise aus einem Pool gespeicherter sinnloser Wörter. Das ausgewählte Wort wird dann der Person, die das Hörhilfegerät trägt (Testperson), über ein Ausgabemedium, vorzugsweise einen Lautsprecher, dargeboten. Ebenso ist aber auch eine drahtgebundene oder drahtlose elektrische Signalübertragung zwischen dem Testsystem und dem Hörhilfegerät möglich.

Für die Beurteilung der Qualität eines Hörhilfegerätes erhält man mit der Darbietung von Sprachsignalen eine zuverlässigere Aussage im Vergleich zur Darbietung von Sinustönen. Damit der Hörhilfegerätetest in unveränderter Form international, insbesondere sprachenunabhängig, durchführbar ist, umfasst das dargebotene Sprachsignal sinnlose Silben, sog. Logatome. Diese können z.B. aus Vokal-Konsonant-Vokal- (aba, aca, ada,...) oder Konsonant-Vokal-Konsonant- (bab, beb, bib, ...) Folgen bestehen. Gegebenenfalls sind diese sinnlosen Silben noch dahingehend zu prüfen und nur solche auszuwählen, die in allen wichtigen Sprachen keinen Sinn ergeben. Die dargebotenen Silben resultieren vorzugsweise aus Sprachaufnahmen. Sie können aber auch synthetisch erzeugt werden.

Eine dargebotene Silbe (z.B. aba) wird schließlich zusammen mit anderen ähnlich klingenden Silben (z.B. ada, aga, aha, aka, ...) auf einer grafischen Benutzeroberfläche des Testsystems dargestellt. Die Testperson wählt aus den dargestellten Silben diejenige aus, z.B. durch ein Zeigerinstrument, die sie meint, gehört zu haben. Alternativ kann die von der Testperson erkannte Silbe von dieser auch nachgesprochen und durch eine Spracherkennung erfasst werden.

Schließlich erfolgt eine Auswertung der getroffenen Auswahl. Im einfachsten Fall wird dadurch die Auswahl als richtig oder falsch gekennzeichnet.

Der Test wird unter erneuter Auswahl sinnloser Silben wiederholt, solange, bis ein Abbruchkriterium erreicht ist.

Gemäß der Erfindung erfolgt die Auswahl des akustischen Sprachsignals nach jedem Durchgang in Abhängigkeit wenigstens einer getroffenen Auswahl der Testperson auf ein zuvor dargebotenes Sprachsignal. Es erfolgt also nach jedem Durchgang eine sofortige Auswertung der von der Testperson auf das Sprachsignal getroffenen Auswahl. Die zuletzt getroffene Auswahl und vorzugsweise auch die Ergebnisse weiter zurückliegender Durchläufe gehen in die Auswahl eines neuen Sprachsignal, insbesondere eines Logatoms, ein. Die Auswahl der Sprachsignale (Logatome) ist somit adaptiv, abhängig von der "Vorgeschichte". Die adaptive Auswahl der Sprachsignale aus dem Sprachsignal-Pool ermöglicht stets die Darbietung der Sprachsignale, die mit der größten Wahrscheinlichkeit Antworten auf die Fragestellungen des aktuellen Tests geben. Sprachsignale, die für den aktuellen Test weniger geeignet sind, werden übersprungen. Dadurch kann die Testdauer reduziert werden, da die Testperson nicht bei jedem Test durch den gesamten Vorrat an Sprachsignalen (Signal-Pool) geführt wird. Durch die gezielte Auswahl der Sprachsignale und die allmähliche Fokusierung der Sprachsignale während des laufenden Tests werden bei der Testperson vorhandene Problembereiche hinsichtlich des Sprachverstehens, z.B. die Fähigkeit, bestimmte Konsonanten richtig zu erkennen, aufgespürt und eingegrenzt.

Bei einer bevorzugten Ausführungsform der Erfindung wird dem dargebotenen Sprachsignal ein Störsignal, insbesondere Rauschen, überlagert. Bei der Darbietung von Sprachsignalen in Störlärm spielt nämlich die Verstärkungseinstellung des Hörhilfegerätes der Testperson eine untergeordnete Rolle. Die Testsignale müssen dann lediglich in einer Lautstärke dargeboten werden, mit der sie gut wahrnehmbar sind. Die Sensitivität des Hörtest ergibt sich aus einer Variation des Signal-Rausch-Verhältnisses (SNR - Signal-Noise-Ratio). Dabei startet der Test zunächst mit keinem oder einem geringen Rauschanteil und der Rauschanteil wird dann relativ zu dem Sprachanteil mit jedem Durchlauf zunehmend vergrößert, solange, bis die Testperson das Sprachsignal nicht mehr richtig erkennt.

Das Verhältnis zwischen Sprachsignal und Störsignal, bei dem die Testperson das Sprachsignal nicht mehr erkennt, erlaubt dann eine zuverlässige Aussage darüber, wie gut das Hörvermögen der Testperson mit einem Hörhilfegerät und damit die Qualität des Hörhilfegerätes bzw. der Versorgung insgesamt ist.

Eine weitere Verbesserung der Zuverlässigkeit bzw. Aussagekraft des Hörhilfegerätetests kann dadurch erreicht werden, dass als Störsignal fluktuierendes Rauschen, insbesondere amplitudenmoduliertes Rauschen mit einer vorzugsweise zufälligen Modulation verwendet wird. Durch die Verwendung eines modulierten Störgeräusches wird eine hohe Sensitivität für eine Hörminderung erreicht, da insbesondere bei einer Innenohrschwerhörigkeit die Schwelle bei modulierten Schallen gegenüber Normalhörenden deutlich angehoben ist.

Besonders zuverlässige und gute Ergebnisse ergeben sich weiterhin dann, wenn das Sprachsignal jeweils im Bereich eines Minimums des fluktuierenden Störsignals dargeboten wird.

Eine Ausführungsform der Erfindung sieht vor, dass nicht nur die Sprachsignale an sich, sondern auch die auf eine Sprachsignal dargebotenen Antwort-Alternativen adaptiv an den bisherigen Testverlauf angepasst werden. Dabei kann der Ähnlichkeitsgrad der dargebotenen Alternativen zu der richtigen Lösung variieren. So können einerseits Alternativen angeboten werden, die einen hohen Ähnlichkeitsgrad aufweisen und andererseits Alternativen, die der korrekten Lösung eher unähnlich sind.

Ebenso wie die Auswahl der Sprachsignale erfolgen auch die Auswahl von Störlärm, die Einstellung der Signalpegel und insbesondere des Pegelverhältnisses zwischen Sprach und Störlärm sowie der Zeitpunkt des Sprachsignals in fluktuierendem Rauschen adaptiv, das heißt in Abhängigkeit der Ergebnisse zuvor erfolgter Test-Durchläufe.

Mit Hilfe des erfindungsgemäßen Verfahrens lässt sich die Verbesserung des Sprachverstehens durch die Verwendung eines Hörhilfegerätes qualitativ und quantitativ erfassen. Zudem ist bei ein und derselben Testperson ein Vergleich zwischen unterschiedlichen Hörhilfegeräten möglich. Weiterhin können bei ein- und demselben Hörhilfegerät unterschiedliche Einstellungen hinsichtlich ihres Einflusses auf das Sprachverstehen untersucht werden, beispielsweise der Einfluss einer Kompression, einer Frequenzkompression oder eines Störgeräuschbefreiungsalgorithmus.

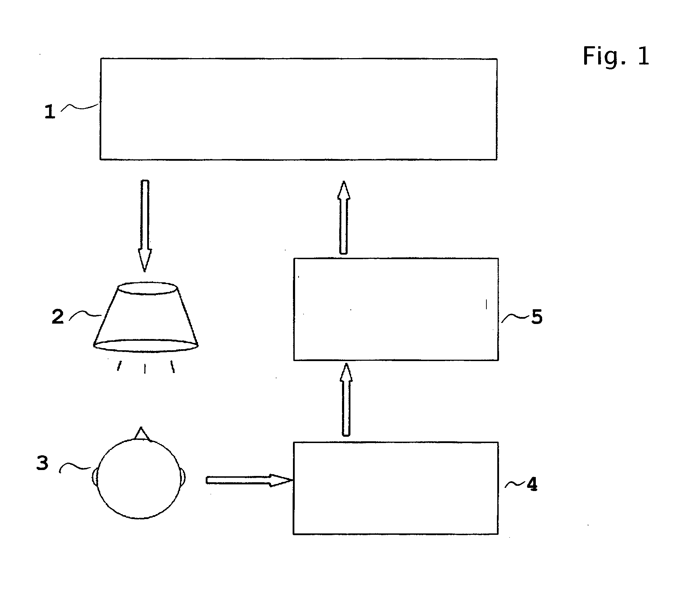

Die Erfindung wird nachfolgend anhand eines Ablaufschemas näher erläutert. Dabei zeigt die Figur die unterschiedlichen Stationen bei der Durchführung eines Hörhilfegeräte-Tests gemäß der Erfindung.

Zunächst wird aus einem Pool von Logatomen 1 zufällig ein Logatom ausgewählt und über einen Lautsprecher 2 einer mit einem Hörhilfegerät versorgten Testperson 3 dargeboten. Weiterhin erhält die Testperson das akustisch dargebotene Logatom zusammen mit ähnlichen Logatomen auf einem Display 4 zur Auswahl angeboten. Die Testperson wählt daraufhin das Logatom aus den auf dem Display angebotenen Logatomen aus, das sie meint, auch auf akustischem Wege dargeboten bekommen zu haben. Die getroffene Auswahl der Testperson wird zunächst in einer Analyse- und Auswahleinrichtung 5 analysiert, insbesondere dahingehend, ob die Auswahl richtig gewesen ist. In Abhängigkeit des Analyseergebnisses erfolgt dann gezielt erneut eine Auswahl eines Logatoms, das der Testperson anschließend dargeboten wird. Somit schließt sich der Kreis und das Verfahren wird so lange fortgeführt, bis ein Abbruchkriterium erreicht ist.

## Patentansprüche

1. Verfahren zum Test des Sprachverstehens einer mit einem Hörhilfegerät versorgten Person mit folgenden Schritten:
a) Bereitstellen eines Testsystems,
b) Auswahl und Darbietung eines akustischen Sprachsignals in Form wenigstens einer sinnlosen Silbe,
c) Darstellung der sinnlosen Silbe und einer Anzahl weiterer sinnloser Silben auf einer grafischen Benutzeroberfläche des Testsystems,
d) Auswahl einer gehörten sinnlosen Silbe aus den dargestellten sinnlosen Silben durch den Benutzer, und
e) Auswertung der getroffenen Auswahl,
**gekennzeichnet durch** den Schritt:
f) Wiederholen der Verfahrensschritte b) bis e), bis ein Abbruchkriterium erreicht ist, wobei die Auswahl des akustischen Sprachsignals jeweils in Abhängigkeit wenigstens einer getroffenen Auswahl der Person auf ein zuvor dargebotenes Sprachsignal erfolgt.

2. Verfahren nach Anspruch 1, wobei dem dargebotenen Sprachsignal ein Störsignal, insbesondere Rauschen, überlagert wird.

3. Verfahren nach Anspruch 2, wobei ein fluktuierendes Störsignal überlagert wird.

4. Verfahren nach Anspruch 3, wobei das Sprachsignal im Bereich eines Minimums des fluktuierenden Störgeräusches dargeboten wird.

5. Verfahren nach einem der Ansprüche 2 bis 4, wobei die Auswahl und/oder Darbietung des akustischen Sprachsignals und/oder des Störsignals zur Wiederholung der Verfahrensschritte b) bis e) in Abhängigkeit der Auswertung der von der Person getroffenen Auswahl bezüglich wenigsten eines zuvor erfolgten Durchlaufes der Verfahrensschritte b) bis e) erfolgt.

6. Verfahren nach Anspruch 5, wobei bei der adaptiven Darbietung des akustischen Sprachsignals und/oder Störsignals das Signal-Rausch-Verhältnis zwischen dem Sprachsignal und dem Störsignal durch Einstellung ihres Pegelverhältnisses angepasst wird.

7. Verfahren nach einem der Ansprüche 1 bis 6, wobei der Ähnlichkeitsgrad zwischen der ausgewählten sinnlosen Silbe und den weiteren sinnlosen Silben angepasst wird.

## Claims

1. Method for testing the speech comprehension of a person assisted by a hearing aid, having the following steps:
a) providing a test system,
b) selecting and presenting an acoustic voice signal in the form of at least one meaningless syllable,
c) displaying the meaningless syllable and a number of further meaningless syllables on a graphical user interface of the test system,
d) the user selecting a heard meaningless syllable from the displayed meaningless syllables,
e) evaluating the selection made,
**characterised by** the step:
f) repeating the method steps b) to e) until a termination criterion is reached, the selection of the acoustic voice signal being made in each instance as a function of at least one selection made by the person in response to a previously presented voice signal.

2. Method according to claim 1, wherein an interference signal, in particular noise, is superimposed on the presented voice signal.

3. Method according to claim 2, wherein a fluctuating interference signal is superimposed.

4. Method according to claim 3, wherein the voice signal is presented in the region of a minimum of the fluctuating noise interference.

5. Method according to one of claims 2 to 4, wherein the selection and/or presentation of the acoustic voice signal and/or of the interference signal to repeat the method steps b)to e) take(s) place as a function of the evaluation of the selection made by the subject in relation to at least one previous round of the method steps b) to e).

6. Method according to claim 5, wherein the signal-to-noise ratio between the voice signal and the interference signal is adjusted by setting its level ratio during the adaptive presentation of the acoustic voice signal and/or of the interference signal.

7. Method according to one of claims 1 to 6, wherein the degree of similarity between the selected meaningless syllable and the further meaningless syllables is adjusted.

## Revendications

1. Procédé de test de la compréhension linguistique d'une personne appareillée d'une prothèse auditive, comprenant les stades suivants :
a) on se procure un système de test,
b) on sélectionne et on présente un signal linguistique acoustique sous la forme d'au moins une syllabe dénuée de sens,
c) on représente la syllabe dénuée de sens et un nombre d'autres syllabes dénuées de sens sur une surface graphique utilisateur du système de test,
d) l'utilisateur sélectionne une syllabe entendue dénuée de sens parmi les syllabes représentées dénuées de sens, et
e) on exploite la sélection manifestée,
**caractérisé par** le stades :
f) on répète les stades b) à e) du procédé jusqu'à obtention d'un critère de rupture, la sélection du signal linguistique acoustique s'effectuant respectivement en fonction d'au moins une sélection manifestée par la personne sur un signal linguistique présenté auparavant.

2. Procédé suivant la revendication 1, dans lequel on superpose un signal parasite, notamment du bruit au signal linguistique présenté.

3. Procédé suivant la revendication 2, dans lequel on superpose un signal parasite fluctuant.

4. Procédé suivant la revendication 3, dans lequel on présente le signal linguistique dans la plage d'un minimum du bruit parasite fluctuant.

5. Procédé suivant l'une des revendications 2 à 4, dans lequel on effectue la sélection et/ou la présentation du signal linguistique acoustique et/ou du signal parasite pour la répétition des stades b) à e) du procédé, en fonction de l'exploitation de la sélection manifestée par la personne en ce qui concerne au moins un déroulement effectué auparavant des stades d) à e) du procédé.

6. Procédé suivant la revendication 5, dans lequel lors de la présentation adaptative du signal linguistique acoustique et/ou du signal parasite, on adapte le rapport signal-bruit entre le signal linguistique et le signal parasite en réglant son rapport de niveau.

7. Procédé suivant l'une des revendications 1 à 6, dans lequel on adapte le degré de similitude entre la syllabe sélectionnée dénuée de sens et les autres syllabes dénuées de sens.
